# EUROPEAN PATENT APPLICATION

(11) **EP 4 484 948 A1**
(43) Date of publication of application: **01.01.2025**
(21) Application number: 24181086.0
(22) Date of filing: 10.06.2024
(51) Int. Cl.: G01N 33/00, G01D 11/24

(54) **A PROTECTIVE SHIELD FOR A SENSOR SUBASSEMBLY**

(30) Priority: 30.06.2023 US 202363511408 P
(71) Applicant: Carrier Corporation, Palm Beach Gardens, FL 33418 (US)
(72) Inventor: HILL, Seth, Syracuse, 13221 (US); PIECH, Marcin, East Hampton, 06424 (US); BIRNKRANT, Michael, Syracuse, 13221 (US); HOVARDAS, Sotiri, Indianapolis, 46231 (US)
(74) Representative: Dehns

(57) **Abstract**

A protective shield (100) for a sensor subassembly (101) includes a cover member (102) and a plurality of mounting tabs (103). The plurality of mounting tabs extend from the cover member. Each of the plurality of mounting tabs is adapted to engage with a sensor housing (105) of the sensor subassembly, such that the cover member is adapted to cover at least one sensor accommodated in the sensor subassembly. The provision of the cover member prevents at least one of heat, ambient particulate matter, particles or spray of liquid that may be present in the HVAC environment (such as water, oil, and refrigerant), vibration, electrical noise, and other emissions within the environment of the HVAC system from directly contacting the at least one sensor accommodated in the sensor housing.

## Description

### CROSS REFERENCE TO RELATED APPLICATION

This application claims the benefit of U.S. Provisional Patent Application No. 63/511,408 filed on June 30, 2023, which is incorporated by reference herein in its entirety.

### FIELD OF THE INVENTION

The invention generally relates to sensor subassemblies. More particularly, the invention relates to a protective shield for a sensor subassembly.

### BACKGROUND

HVAC systems utilize refrigerants within a closed loop circuit to condition air provided to an area or enclosed space. This refrigerant, historically, has been provided as a fluid with a high global warming potential (GWP) value such as R134A or R410A. Thus, although the refrigerants that have been used previously are effective coolants, the negative effect they can have on the environment has led to regulatory requirements to transition to refrigerants which have moderate-to-low GWP values.

Generally, modern refrigerants comply with environmental regulations relating to global warming potential (GWP). To comply with the proposed GWP regulations, hydrofluorocarbon (HFC) and hydrocarbon refrigerants with various levels of flammability are being developed and considered for use in the HVAC system. This has necessitated the use of sensors and sensor subassemblies.

Typically, sensor subassemblies are widely used to accommodate sensors that detect the presence of unforeseen ambient conditions, for example, leakage of refrigerants, presence of toxic gases, a fire, and the like in industrial, commercial, and residential settings. To protect the sensors from environmental conditions, physical damage, or other factors that may affect their performance, several protective shields have been developed over the years.

However, despite their utility, existing protective shields are not without disadvantages. One major issue is cost as periodic maintenance of protective shields is desirable to ensure optimal functioning of the sensor subassembly. This can be time consuming and costly, especially in industrial settings. Similarly, installation of existing protective shields can be difficult, especially in older buildings or other challenging environments. Therefore, the existing protective shields can be expensive and cumbersome to install.

Another disadvantage of existing protective shields is that although they protect sensors, the sensitivity of the sensors may be limited. This can make it more difficult to detect gas leaks or other hazardous conditions thereby compromising the safety of the workers or residents. Alternatively, the existing protective shields may cause false alarms, leading to unnecessary evacuations or other safety measures. This can disrupt business operations and lead to confusion or concern among workers or residents.

Weather resistance and susceptibility to electromagnetic interference is another issue with existing protective shields. Some protective shields may not be weather resistant, making the sensors susceptible to damage from extreme temperatures, rain, wind, or other environmental conditions. Similarly, the existing protective shields may not be equipped to protect the sensors from electromagnetic interference. This may cause errors in the functioning of the sensors thereby compromising the reliability of the gas detectors over time.

Therefore, a protective shield for sensors that is weather resistant, easy to install, less susceptible to electromagnetic interference, while remaining cost-effective to implement, is therefore desirable. As such, the protective shield that overcomes these limitations and provides improved protection for the sensors while maintaining their sensitivity and accuracy, is therefore desirable.

### SUMMARY

This summary is provided to introduce a selection of concepts in a simplified format that are further described in the detailed description of the invention. This summary is not intended to identify key or essential inventive concepts of the invention, nor is it intended for determining the scope of the invention.

A first aspect of the invention provides a protective shield for a sensor subassembly. The protective shield includes a cover member and a plurality of mounting tabs extending from the cover member. Each of the plurality of mounting tabs are adapted to engage with a sensor housing of the sensor subassembly, such that the cover member is adapted to cover at least one sensor accommodated in the sensor subassembly.

Optionally, the plurality of mounting tabs are engaged with at least one lip portion defined on an outer peripheral surface of the sensor housing.

Optionally, the plurality of mounting tabs is adapted to slidably engage with the at least one lip portion defined on the outer peripheral surface of the sensor housing of the sensor subassembly to restrict the movement of the cover member relative to the sensor housing.

Optionally, each of the plurality of mounting tabs extends from a bottom peripheral surface of the cover member in an orthogonal orientation relative to the cover member.

Optionally, the bottom peripheral surface of the cover member facing the sensor housing, the sensor housing, and the plurality of mounting tabs collectively define a plurality of airflow channels.

Optionally, one or more parameters of the plurality of airflow channels are adjusted by modifying at least one of a length of each of the plurality of mounting tabs, a thickness of each of the plurality of mounting tabs, and a total number of the plurality of mounting tabs.

Optionally, each of the plurality of mounting tabs are equidistant.

Optionally, a first set of mounting tabs from the plurality of mounting tabs abuts at least one lip portion of the sensor housing and a second set of mounting tabs from the plurality of mounting tabs engage with at least one lip portion defined on the outer peripheral surface of the sensor housing.

Optionally, a plurality of grooves is defined on a bottom peripheral surface of the cover member facing the sensor housing.

Optionally, the cover member is made of a heat-reflective material.

A second aspect of the invention provides a sensor subassembly including a sensor housing and a protective shield. The sensor housing is adapted to enclose at least one sensor, at least one internal heater, and control circuitry electrically connected to the at least one sensor and the at least one internal heater. The protective shield, adapted to be removably fastened to the sensor housing, includes a cover member and a plurality of mounting tabs. The plurality of mounting tabs extend from the cover member. Each of the plurality of mounting tabs are adapted to engage with the sensor housing, such that the cover member is adapted to cover the at least one sensor.

Optionally, the plurality of mounting tabs are engaged with at least one lip portion defined on an outer peripheral surface of the sensor housing.

Optionally, the plurality of mounting tabs is adapted to slidably engage with the at least one lip portion defined on the outer peripheral surface of the sensor housing of the sensor subassembly to restrict the movement of the cover member relative to the sensor housing.

Optionally, each of the plurality of mounting tabs extends from a bottom peripheral surface of the cover member in an orthogonal orientation relative to the cover member.

Optionally, the bottom peripheral surface of the cover member facing the sensor housing, the sensor housing, and the plurality of mounting tabs collectively define a plurality of airflow channels.

Optionally, one or more parameters of the plurality of airflow channels are adjusted by modifying at least one of a length of each of the plurality of mounting tabs, a thickness of each of the plurality of mounting tabs, and a number of the plurality of mounting tabs.

Optionally, each of the plurality of mounting tabs are equidistant.

Optionally, a first set of mounting tabs from the plurality of mounting tabs abuts at least one lip portion of the sensor housing and a second set of mounting tabs from the plurality of mounting tabs engage with the at least one lip portion defined on the outer peripheral surface of the sensor housing.

Optionally, a plurality of grooves is defined on a bottom peripheral surface of the cover member facing the sensor housing.

Optionally, the cover member is made of a heat-reflective material.

To further clarify the advantages and features of the systems, and apparatuses, a more particular description of the systems, and apparatuses will be rendered by reference to specific embodiments thereof, which is illustrated in the appended drawings. It is appreciated that these drawings depict only typical embodiments of the invention and are therefore not to be considered limiting its scope. The invention will be described and explained with additional specificity and detail with the accompanying drawings.

### BRIEF DESCRIPTION OF THE DRAWINGS

These and other features, aspects, and advantages of the invention will become better understood when the following detailed description is read with reference to the accompanying drawings in which like characters represent like parts throughout the drawings, wherein:
**FIG. 1** illustrates a bottom perspective isometric view of a sensor subassembly;
**FIG. 2A** illustrates a top perspective view of a protective shield;
**FIG. 2B** illustrates a front planar view of the protective shield;
**FIG. 2C** illustrates a top planar view of the protective shield; and
**FIG. 2D** illustrates a bottom planar view of the protective shield.

Further, skilled artisans will appreciate that elements in the drawings are illustrated for simplicity and may not have necessarily been drawn to scale.. Furthermore, in terms of the construction of the device, one or more components of the device may have been represented in the drawings by conventional symbols, and the drawings may show only those specific details that are pertinent to understanding the embodiments of the invention so as not to obscure the drawings with details that will be readily apparent to those of ordinary skill in the art having the benefit of the description herein.

### DETAILED DESCRIPTION

For the purpose of promoting an understanding of the principles of the invention, reference will now be made to the various embodiments and specific language will be used to describe the same. It will nevertheless be understood that no limitation of the scope of the invention is thereby intended, such alterations and further modifications in the illustrated system, and such further applications of the principles of the invention as illustrated therein being contemplated as would normally occur to one skilled in the art to which the invention relates.

It will be understood by those skilled in the art that the foregoing general description and the following detailed description are explanatory of the invention and are not intended to be restrictive thereof.

Reference throughout this specification to "an aspect", "another aspect" or similar language means that a particular feature, structure, or characteristic described in connection with the embodiment is included in at least one embodiment of the invention. Thus, appearances of the phrase "in an embodiment", "in another embodiment", "some embodiments", "one or more embodiments" and similar language throughout this specification may, but do not necessarily, all refer to the same embodiment.

The terms "comprises", "comprising", or any other variations thereof, are intended to cover a non-exclusive inclusion, such that a process or method that comprises a list of steps does not include only those steps but may include other steps not expressly listed or inherent to such process or method. Similarly, one or more devices or sub-systems or elements or structures or components proceeded by "comprises... a" does not, without more constraints, preclude the existence of other devices or other sub-systems or other elements or other structures or other components or additional devices or additional sub-systems or additional elements or additional structures or additional components.

Embodiments of the invention will be described below in detail with reference to the accompanying drawings.

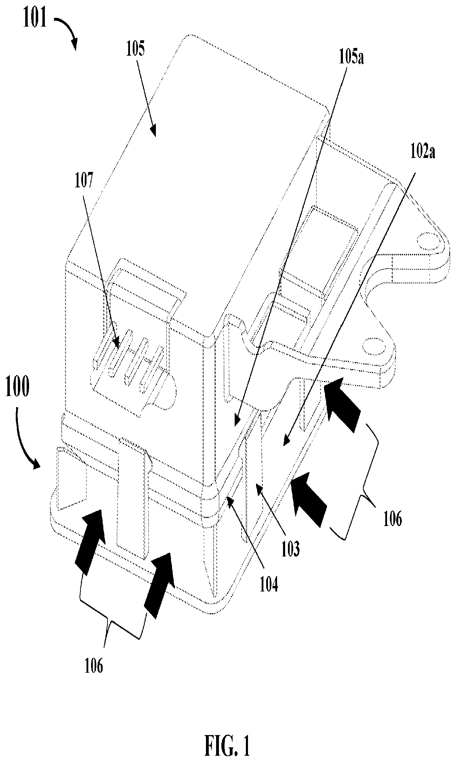

**FIG. 1** exemplarily illustrates a bottom perspective isometric view of a sensor subassembly 101 according to one or more embodiments of the invention. The sensor subassembly 101 includes a protective shield 100 and a sensor housing 105 such that the protective shield 100 is adapted to be removably fastened to the sensor housing 105.

In an embodiment, the sensor subassembly 101 may be employed in a Heating, Ventilating, and Air Conditioning System (HVAC) system to detect a refrigerant leak therein. The provision of the sensor subassembly 101 enables quick detection of leaks, for example, within ten seconds of being exposed to a 100% lower flammability limit (LFL), within thirty seconds of being exposed to a 25% lower flammability limit (LFL), etc. A lower flammability limit (LFL) of the refrigerant is the minimum concentration limit that is desired for the refrigerant to become potentially combustible. In an embodiment, the sensor subassembly 101 described herein may be capable of detecting a leak of an A2L refrigerant when the sensor subassembly 101 detects at least 5 to 50% LFL in the sample. In an embodiment, the refrigerant circulating through the HVAC system 20 is the A2L refrigerant. The A2L refrigerant is a classification of refrigerant based upon American Society of Heating, Refrigerating and Air-Conditioning (ASHRAE) Standard 34. The standard evaluates each refrigerant's flammability and toxicity and gives it a class referenced as a letter and number combination. The first letter refers to the refrigerants' toxicity and is based on the refrigerant's occupational exposure limit (OEL). The number adjacent to the letter refers to the refrigerants' flammability and is based on the burning velocity (BV), heat of combustion (HOC), and lower flammability limits (LFL) of the refrigerant.

With continued reference to **FIG. 1****,** in rare instances the refrigerant within a closed loop refrigerant circuit of the HVAC system may leak leading to undesirable consequences due to the mildly flammable nature of the refrigerant. The sensor subassembly 101, using at least one sensor, performs the function of early detection of a leak to prevent the undesirable consequences of a prolonged or excessive leak of the refrigerant. In an embodiment, the sensor housing 105 of the sensor subassembly 101 is adapted to enclose the at least one sensor, at least one internal heater, and control circuitry electrically connected to the at least one sensor and the at least one internal heater. In an embodiment, the at least one sensor, the at least one internal heater, and the control circuitry may be positioned at least partially or wholly within a cavity (not shown) defined within the sensor housing 105. As such, the cover member 102 is adapted to cover the at least one sensor at least partially or wholly.

In an embodiment, the surface of the cover member 102 facing the sensor housing 105, the sensor housing 105, and the plurality of mounting tabs 103 collectively define a plurality of airflow 106 channels for receiving the airflow 106 from outside the sensor subassembly 101. The enhanced airflow 106 into the sensor housing 105 ensures improved response time of the at least one sensor. Furthermore, the life of the at least one sensor and the control circuitry is improved due to protection from extreme heat or ambient temperatures. In an embodiment, one or more parameters of the plurality of airflow 106 channels are adjusted by modifying at least one of a length of each of the plurality of mounting tabs 103, a thickness of each of the plurality of mounting tabs 103, and a total number of the plurality of mounting tabs 103. As used herein, the term "one or more parameters" refer to a volume of each of the plurality of airflow 106 channels, an airflow 106 rate associated with each of the plurality of the airflow 106 channels, etc. For example, by increasing the length of each of the plurality of mounting tabs 103, the total volume of the airflow 106 into the sensor housing 105 may be increased. In another implementation, by increasing the number of mounting tabs 103, the number of airflow 106 channels are increased. It may be appreciated that several adjustments to the geometrical dimensions and arrangement of the plurality of mounting tabs 103 may be envisioned by those skilled in the art without departing from the scope of the invention.

In an embodiment, the at least one sensor is a non-dispersive infrared (NDIR) sensor including an infra-red light source and at least one corresponding detector element. As is known in the art, a NDIR sensor is configured to use light, for example infrared light, to evaluate absorption characteristics of gas molecules within a light path. The gas molecules are identified, and the concentration of the gas molecules may be determined by utilizing a relation (Lambert-Beer law) between a gas concentration and an absorption intensity. The at least one sensor is configured to detect the presence of refrigerant molecules, such as an A2L refrigerant. Accordingly, when a leak is present, a sample of air at the at least one sensor will contain a mixture of air and refrigerant. Since the at least one sensor periodically samples the air within the HVAC system, the sensitivity of the at least one sensor is desirably protected using the protective shield 100. The protective shield 100 isolates the at least one sensor from at least one of heat, ambient particulate matter, particles or spray of liquid that may be present in the HVAC environment (such as water, oil, and refrigerant), vibration, electrical noise, and other emissions within the environment of the HVAC system thereby preventing loss of sensitivity or damage to the at least one sensor.

The control circuitry may include a printed circuit board disposed generally adjacent to a portion, such as the bottom surface, of the at least one sensor. In an embodiment, the control circuitry may also include other components associated with an operation of the at least one sensor, for example, the light source, the detector element, etc. The control circuitry may also include signal amplification and conditioning electronics. As used herein, the term "control circuitry" may be construed to encompass one or a combination of microprocessors, suitable logic, circuits, printed circuit boards (PCB), audio interfaces, visual interfaces, haptic interfaces, or the like. The control circuitry may include, but is not limited to, a microcontroller, a Reduced Instruction Set Computing (RISC) processor, an Application-Specific Integrated Circuit (ASIC) processor, a Complex Instruction Set Computing (CISC) processor, a central processing unit (CPU), a graphics processing unit (GPU), a state machine, and/or other processing units or circuits.

In an embodiment, the heat generated by the infra-red light source of the at least one sensor and/or the other electrical components associated with or powered by the control circuitry may be sufficient to maintain the at least one sensor at a temperature above ambient to prevent condensation and/or frosting. However, in other embodiments, the control circuitry may include at least one internal heater, for example, resistive heaters as active heating elements embedded therein. In an embodiment, the sensor subassembly 101 may also include any suitable insulating material, such as a rigid foam, for enclosing the at least one sensor and the control circuitry. The insulating material may include one or more openings to permit airflow 106 entry and exit for sampling by the at least one sensor. In an embodiment, the insulating material reduces the gas transport path and increases the robustness of the at least one sensor to frost and condensing water droplets.

The protective shield 100 and the sensor housing 105 of the sensor subassembly 101 collectively increase robustness, reliability, and performance of the sensor subassembly 101 during operation of the HVAC system. In an embodiment, the sensor subassembly 101 is configured to mechanically or chemically integrate or couple to one or more components of the HVAC system, for example, using one or more mounting holes (not shown) formed in the sensor housing 105, an adhesive, etc. Further, the sensor subassembly 101 may be configured to electrically integrate with the HVAC system via one or more connectors 107 formed in the sensor housing 105. In an embodiment, the one or more connectors 107 may be adapted to prevent incorrect electrical integration with the HVAC system. For example, the one or more connectors 107 may include a plastic protrusion that is shaped to match an electrical wire harness used for integration.

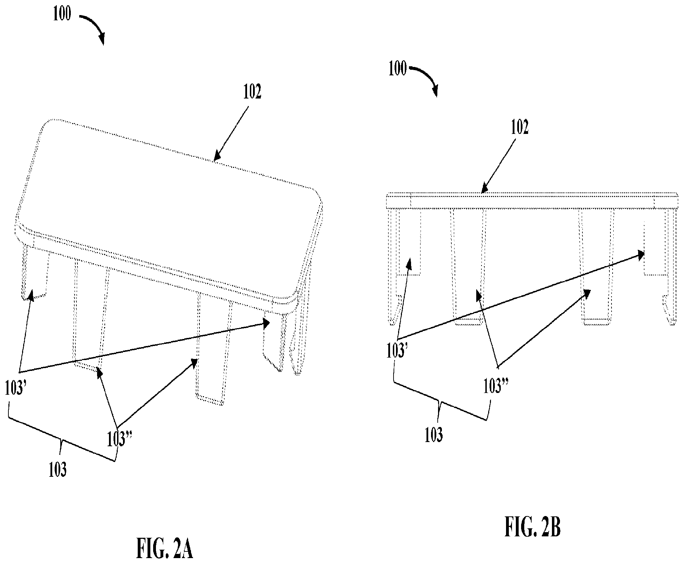

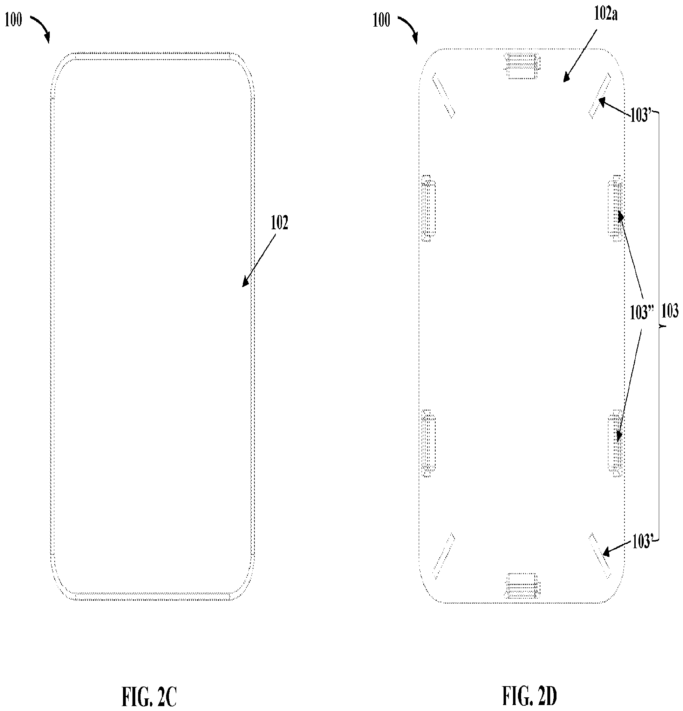

**FIG. 2A** exemplarily illustrates a top perspective view of the protective shield 100 according to one or more embodiments of the invention. **FIG. 2B** exemplarily illustrates a front planar view of the protective shield 100 according to one or more embodiments of the invention. **FIG. 2C** exemplarily illustrates a top planar view of the protective shield 100 according to one or more embodiments of the invention. **FIG. 2D** exemplarily illustrates a bottom planar view of the protective shield according to one or more embodiments of the invention. The protective shield 100 is adapted to be removably fastened to the sensor housing 105 exemplarily illustrated in **FIG. 1****.** The protective shield 100 includes a cover member 102 and a plurality of mounting tabs 103 extending from the cover member 102. In an embodiment, each of the plurality of mounting tabs 103 is adapted to engage with at least one lip portion 104 defined on an outer peripheral surface 105a of the sensor housing 105 to restrict a movement of the cover member 102 relative to the sensor housing 105.

In an embodiment, each of the plurality of mounting tabs 103 are snap fit or clip fit onto the at least one lip portion 104 of the sensor housing 105. Alternatively, each of the plurality of mounting tabs 103 is adapted to slidably engage with the at least one lip portion 104 defined on the outer peripheral surface 105a of the sensor housing 105 to restrict the movement of the cover member 102 relative to the sensor housing 105. In an embodiment, each of the plurality of mounting tabs 103 extends from a bottom peripheral surface 102a of the cover member 102 in an orthogonal orientation relative to the cover member 102. In an embodiment, each of the plurality of mounting tabs 103 is equidistant. In another embodiment, each of the plurality of mounting tabs 103 is not equidistant. In an embodiment, a first set of mounting tabs 103' from the plurality of mounting tabs 103 abuts the at least one lip portion 104 of the sensor housing 105 and a second set of mounting tabs 103" from the plurality of mounting tabs 103 engage with the at least one lip portion 104 defined on the outer peripheral surface 105a of the sensor housing 105.

In an embodiment, the first set of mounting tabs 103' are positioned at four corners of the bottom peripheral surface 102a of the cover member 102 as shown in **FIG. 2D****.** The provision of the first set of mounting tabs 103' at the four corners prevents erratic movement of the cover member 102 relative to the sensor housing 105. Simultaneously, the second set of mounting tabs 103"engage with the at least one lip portion 104 to further secure the cover member 102 to the sensor housing 105. Therefore, the provision of the first set of mounting tabs 103' and the second set of mounting tabs 103" ensure a secure fit between the cover member 102 and the sensor housing 105.

With continued reference to FIG. 1A, the protective shield 100 functions to isolate the at least one sensor from heat, ambient particulate matter, particles or spray of liquid that may be present in the HVAC environment (such as water, oil and refrigerant), vibration, electrical noise, and other emissions within the environment of the HVAC system. The protective shield 100 may further prevent the accumulation of frost or condensation on the at least one sensor. In an embodiment, the cover member 102 may also electromagnetically shield the at least one sensor from electromagnetic interference. Moreover, the cover member 102 is made of a heat-reflective material for dissipating heat. The cover member 102 is oriented to prevent at least one of heat, ambient particulate matter, and water particles from contacting the at least one sensor accommodated in the sensor housing 105. This means that based on the location of the sensor subassembly 101 in the HVAC system and an airflow 106 within the HVAC system, the cover member 102 may be positioned such that optimal prevention of heat, ambient particulate matter, and particles or spray of liquid that may be present in the HVAC environment (such as water, oil, and refrigerant) from directly striking the at least one sensor is achieved. This prolongs the life of the at least one sensor and prevents loss of sensitivity or damage to the at least one sensor.

In an embodiment, the cover member 102 may also include a plurality of grooves (not shown) defined on the bottom peripheral surface 102a (shown in **FIG. 1****,** **2D**) of the cover member 102 facing the sensor housing 105. The provision of the plurality of grooves and other airflow 106 enhancing structures, for example, scoop-shaped inlets, etc. allow improved ingress of airflow 106 into the sensor housing 105 of the sensor subassembly 101 as exemplarily illustrated in FIG. 1A. In an embodiment, the surface of the cover member 102 facing the sensor housing 105, the sensor housing 105, and the plurality of mounting tabs 103 collectively define a plurality of airflow channels for receiving the airflow 106 from outside the sensor subassembly 101. The enhanced airflow 106 into the sensor housing 105 ensures improved response time of the at least one sensor. Furthermore, the life of the at least one sensor and the control circuitry is improved due to protection from extreme heat or ambient temperatures.

The protective shield 100 is adapted to facilitate enhanced airflow 106 into the sensor subassembly 101 accommodating the at least one sensor for detecting the refrigerant leak in the HVAC system or furnace that has transient temperature changes. In addition to facilitating enhanced airflow 106, the protective shield 100 also functions to isolate the at least one sensor from heat, ambient particulate matter, particles or spray of liquid that may be present in the HVAC environment (such as water, oil, and refrigerant), vibration, electrical noise, oil particles, refrigerant particles, and other emissions within the environment of the HVAC system.

The provision of the internal heater prevents the accumulation of frost or condensation on the at least one sensor. Moreover, the provision of the insulating material reduces the gas transport path and increases the robustness of the at least one sensor to frost and condensing water droplets.

In an embodiment, the cover member 102 of the protective shield 100 may also electromagnetically shield the at least one sensor from electromagnetic interference. Moreover, the cover member 102 is made of a heat-reflective material for dissipating heat and serves to deflect heat from the environment thereby shielding or better protecting the at least one sensor housed within the sensor housing 105. Additionally, the cover member 102 is oriented to prevent at least one of heat, ambient particulate matter, particles or spray of liquid that may be present in the HVAC environment (such as water, oil, and refrigerant), vibration, electrical noise, and other emissions within the environment of the HVAC system from contacting the at least one sensor accommodated in the sensor housing 105. The cover member 102 also protects the at least one sensor from a direct hit of oil/refrigerant spray should a leak occur in the vicinity of the at least one sensor.

The snap fit or clip fit or slidable engagement of the cover member 102 allows the cover member 102 to be easily assembled and/or dismantled thereby improving ease of replacement. Moreover, in the event of repair or maintenance of the sensor subassembly 101, the cover member 102 may be easily removed to facilitate access to the interior of the sensor subassembly 101. Moreover, since the cover member 102 is spaced apart from the sensor housing 105, enhanced airflow 106 is facilitated. In an embodiment, the cover member 102 may be made from a heat absorbent plastic but can be made from other materials/coatings having improved heat reflecting characteristics. The cover member 102 may be manufactured using a 3D printing method or a conventional plastic manufacturing method, for example, an injection molding method and the like.

As used herein, the term "control circuitry" may also include suitable logic, circuits, interfaces, and/or code that may be configured to execute a set of instructions stored in a memory unit. In an exemplary implementation of the memory unit according to the invention, the memory unit may include, but is not limited to, Electrically Erasable Programmable Read-only Memory (EEPROM), Random Access Memory (RAM), Read Only Memory (ROM), Hard Disk Drive (HDD), Flash memory, Solid-State Drive (SSD), and/or CPU cache memory.

In an exemplary embodiment, the control circuitry may receive power from a suitably coupled power source (not shown). For example, a battery or a power source may be electrically coupled to supply electrical power to the control circuitry. In an embodiment, the power source may be, for example, a battery, such as a rechargeable battery or a non-rechargeable battery. Examples of suitable batteries include, for example, a lithium battery (such as a lithium-ion battery), a nickel battery (such as a nickel-cadmium battery), and an alkaline battery.

The control circuitry may also include a communication unit adapted to communicate with other sensor subassemblies of the HVAC detection system or a hazard detection system. In an embodiment, the communication unit also transmits data to and receives data from other sensor subassemblies or components of the HVAC system via a communication network. The communication unit may be configured of, for example, a telematic transceiver (DCM), a mayday battery, a GPS, a data communication module ASSY, a telephone microphone ASSY, and a telephone antenna ASSY. The communication network may include, but is not limited to, a Wide Area Network (WAN), a cellular network, such as a 3G, 4G, or 5G network, an Internet-based mobile ad hoc networks (IMANET), etc. The communication network may also include wired media such as a wired network or direct-wired connection, and wireless media such as acoustic, radio frequency (RF), microwave, infrared (IR), and other wireless media.

In an embodiment, the communication unit may also transmit data to and receive data from a computing device via a communication network. In an embodiment, the computing device may be implemented as a portion of a small-form factor portable (or mobile) electronic device such as a cell phone, a personal data assistant (PDA), a personal media player device, a wireless web-watch device, a personal headset device, an application specific device, or a hybrid device that include any of the above functions. In an embodiment, the control circuitry is further configured to generate a secondary notification on the computing device based on the control circuitry determining presence of the leakage of the refrigerant.

In some exemplary implementations according to the invention, the computing device may be adapted to generate a visual notification in addition to the audio notification via a display interface of the computing device. The display may include suitable logic, circuitry, interfaces, and/or code that may be configured to render several types of information and/or entertainment content via a user interface. In an embodiment, the display may be a flashing visual indicator, such as a Light Emitting Diode (LED), indicator lights, or the like. The user interface may be a customized Graphic User Interface (GUI) configured to display information related to the HVAC system. The display may include, but is not limited to, a projection-based display, an electro-chromic display, a flexible display, and a holographic display. In other embodiments, the display may be a touchscreen display, a tactile electronic display, and/or a touchable hologram.

While specific language has been used to describe the subject matter, any limitations arising on account thereto, are not intended. As would be apparent to a person in the art, various working modifications may be made to the apparatuses in order to implement the inventive concept as taught herein. The drawings and the foregoing description give examples of embodiments. Those skilled in the art will appreciate that one or more of the described elements may well be combined into a single functional element. Alternatively, certain elements may be split into multiple functional elements. Elements from one embodiment may be added to another embodiment.

## Claims

1. A protective shield (100) for a sensor subassembly (101), the protective shield comprising:
a cover member (102); and
a plurality of mounting tabs (103) extending from the cover member, each of the plurality of mounting tabs adapted to engage with a sensor housing (105) of the sensor subassembly, wherein the cover member is adapted to cover at least one sensor accommodated in the sensor subassembly.

2. The protective shield according to claim 1, wherein the plurality of mounting tabs (103) are engaged with at least one lip portion (104) defined on an outer peripheral surface (105a) of the sensor housing (105).

3. The protective shield according to claim 2, wherein the plurality of mounting tabs (103) is adapted to slidably engage with the at least one lip portion (104) defined on the outer peripheral surface (105a) of the sensor housing (105) of the sensor subassembly (101) to restrict a movement of the cover member (102) relative to the sensor housing (105).

4. The protective shield according to claim 1, wherein a first set of mounting tabs (103') from the plurality of mounting tabs abuts at least one lip portion (104) of the sensor housing (105) and a second set of mounting tabs (103") from the plurality of mounting tabs engage with the at least one lip portion (104) defined on an outer peripheral surface (105a) of the sensor housing (105).

5. The protective shield according to any preceding claim, wherein each of the plurality of mounting tabs (103) extends from a bottom peripheral surface (102a) of the cover member (102) in an orthogonal orientation relative to the cover member.

6. The protective shield according to any preceding claim, wherein a bottom peripheral surface (102a) of the cover member (102) facing the sensor housing, the sensor housing (105), and the plurality of mounting tabs (103) collectively define a plurality of airflow channels (106).

7. The protective shield according to claim 6, wherein one or more parameters of the plurality of airflow channels (106) are adjusted by modifying at least one of a length of each of the plurality of mounting tabs (103), a thickness of each of the plurality of mounting tabs, and a number of the plurality of mounting tabs.

8. The protective shield according to any preceding claim, wherein each of the plurality of mounting tabs (103) are equidistant.

9. The protective shield according to any preceding claim, wherein a plurality of grooves is defined on a bottom peripheral surface (102a) of the cover member (102) facing the sensor housing (105).

10. The protective shield according to any preceding claim, wherein the cover member (102) is made of a heat-reflective material.

11. A sensor subassembly (101) comprising:
a sensor housing (105) adapted to enclose at least one sensor, at least one internal heater, and control circuitry electrically connected to the at least one sensor and the at least one internal heater; and
the protective shield (100) as claimed in any preceding claim, wherein the protective shield is adapted to be removably fastened to the sensor housing.
